# EUROPEAN PATENT APPLICATION

(11) **EP 4 159 139 A1**
(43) Date of publication of application: **05.04.2023**
(21) Application number: 21201595.2
(22) Date of filing: 08.10.2021
(51) Int. Cl.: A61B 8/00, A61B 8/08, G06T 7/00

(54) **SYSTEM AND METHOD FOR SEGMENTING AN ANATOMICAL STRUCTURE**

(30) Priority: 30.09.2021 US 202163250468 P
(71) Applicant: Koninklijke Philips N.V., 5656 AG Eindhoven (NL)
(72) Inventor: WEBER, Frank Michael, Eindhoven (NL); MERAL, Faik Can, Eindhoven (NL); WAECHTER-STEHLE, Irina, Eindhoven (NL); VIGNON, Francois Guy Gerard Marie, Eindhove (NL); LOSSAU, Tanja, Eindhoven (NL); NGUYEN, Man M, Eindhoven (NL); WILD, Sebastian, Eindhoven (NL); YU, Jason Richard, Eindhoven (NL); GOOßEN, André, Eindoven (NL); GROTH, Alexandra, Eindhoven (NL)
(74) Representative: Philips Intellectual Property & Standards

(57) **Abstract**

A system and method for segmenting an anatomical structure in ultrasound imaging data. First ultrasound imaging data of a first region, which includes the anatomical structure, is acquired at a high resolution. Second ultrasound imaging data of a second region, which includes a region neighboring the first region, is acquired at a low resolution. The first and second ultrasound imaging data are processed to segment the anatomical structure.

## Description

### FIELD OF THE INVENTION

The invention relates to the field of segmenting anatomical structures, and in particular to the automatic segmentation of anatomical structures in ultrasound images.

### BACKGROUND OF THE INVENTION

Ultrasound imaging data provides useful information for a number of clinical applications. Automated image processing algorithms, such as segmentation algorithms, are often used to process ultrasound imaging data to identify anatomical structures in the images.

A high frame rate is often desirable when obtaining ultrasound imaging data. Achieving a high frame rate is particularly difficult in applications using three-dimensional ultrasound data, such as three-dimensional cardiac ultrasound, since scanning a 3D volume takes considerably longer than scanning a 2D plane of the same object. In order to achieve high frame rates, clinicians are trained to acquire ultrasound imaging data with small fields-of-view. Clinicians continue to acquire images with small fields-of-view even when using more modern ultrasound probes that allow higher frame rates.

However, automated segmentation algorithms, such as HeartModel and FastModel for AutoView developed by Philips Healthcare, typically require a wider image context than these small fields-of-view allow in order to ensure a robust initialization (e.g. for mitral valve segmentation).

One possible solution is to first acquire a larger image (e.g. a full volume image of the left heart) which can be used by the segmentation algorithm to establish anatomical context. Larger images may be acquired intermittently when acquiring small field-of-view images in order to re-ensure the anatomical context. However, this introduces a time delay which may disturb the acquisition flow of the small field-of-view images.

There is therefore a need for an improved method for providing anatomical context for ultrasound images while maintaining a high frame rate.

### SUMMARY OF THE INVENTION

The invention is defined by the claims.

According to examples in accordance with an aspect of the invention, there is provided a processing system for segmenting an anatomical structure in ultrasound imaging data.

The processing system is configured to: obtain first ultrasound imaging data representative of a region of interest including the anatomical structure, wherein the first ultrasound imaging data is acquired at a first resolution; obtain second ultrasound imaging data representative of an extended field-of-view, wherein the second ultrasound imaging data is acquired at a second, lower resolution, and the extended field-of-view comprises at least one region adjacent to the region of interest; and segment the anatomical structure in the region of interest by processing the first ultrasound imaging data and second ultrasound imaging data.

The region of interest is a region with a relatively small field-of-view. This region is imaged at a high resolution for display.

The extended field-of-view provides additional anatomical context to improve an accuracy and reliability of the segmentation of the anatomical structure. In some examples, the extended field-of-view may comprise only the at least one region adjacent to the region of interest. In other examples, the extended field-of-view may comprise at least part of the region of interest and at least one region adjacent to the region of interest. For instance, the extended field-of-view may be a larger region that encompasses the region of interest. In yet other examples, the extended field-of-view may comprise at least one region adjacent to the region of interest and part of the region of interest. For instance, the extended field-of-view may be a region that includes part of the region of interest and one or more regions adjacent to the region of interest.

The inventors have recognized that acquiring ultrasound imaging data for the region of interest at a high resolution and ultrasound imaging data for the extended field-of-view at a low resolution produces sufficient anatomical context for a segmentation algorithm while also allowing a high frame rate.

The processing system may segment the anatomical structure using any suitable segmentation algorithm (e.g. a model-based segmentation algorithm or an AI-based segmentation algorithm).

The first and second ultrasound imaging data may be three-dimensional ultrasound imaging data, for which a high frame rate is particularly desirable. The first and second ultrasound imaging data may be cardiac ultrasound imaging data, in which a high frame is required in order to obtain useful information about cardiac function.

In some examples, the processing system is further configured to: obtain a definition of the region of interest; and define the extended field-of-view based on the received definition of the region of interest.

This allows a suitable extended field-of-view to be automatically defined for a given region of interest. The received definition of the region of interest may be based on user input.

In some examples, the processing system is further configured to control a beamforming unit to: acquire first ultrasound imaging data representative of the region of interest at the first resolution; and acquire second ultrasound imaging data representative of the extended field-of-view at the second, lower resolution.

In some examples, the processing system is configured to: control the beamforming unit to acquire the first ultrasound imaging data; generate an image of the region of interest based on the first ultrasound imaging data; output the generated image of the region of interest to a user interface; and control the beamforming unit to acquire the second ultrasound imaging data in response to receiving a user input.

In this way, an extended field-of-view may only be imaged when a user determines that the region of interest corresponds to a desired field-of-view (and shows, via the user input, an intention for segmenting an anatomical structure in the region of interest).

In some examples, the processing system is configured to: control the beamforming unit to acquire the first ultrasound imaging data; process the first ultrasound imaging data to determine one or more time-dependent anatomical characteristics; determine a time at which to acquire the second ultrasound imaging data based on the determined one or more time-dependent anatomical characteristics; and control the beamforming unit to acquire the second ultrasound imaging data at the determined time.

In this way, the extended field-of-view can be imaged automatically.

For example, in the case of cardiac ultrasound, the second ultrasound imaging data may be acquired at a particular point in the cardiac cycle (e.g. at end-diastole or end-systole).

In some examples, the processing system is configured to: obtain, from one or more sensors, sensor data representative of the anatomical structure; process the sensor data to determine one or more time-dependent anatomical characteristics; determine a time at which to acquire the second ultrasound imaging data based on the determined one or more time-dependent anatomical characteristics; and control the beamforming unit to acquire the second ultrasound imaging data at the determined time.

In some ultrasound imaging applications, the time-dependent characteristics on which the time to acquire the second ultrasound imaging data is based may not be capable of being determined with a desired accuracy from the first ultrasound imaging data. Using data from one or more other sensors may allow the time-dependent characteristics, and therefore the time at which the second ultrasound imaging data should be acquired (e.g. a particular point in the cardiac cycle), with a greater accuracy.

For example, where the first and second ultrasound imaging data are cardiac ultrasound imaging data, the one or more sensors may comprise an ECG sensor. ECG sensor data may be used to determine when a particular point in the cardiac cycle will occur.

The processing system may control the beamforming unit to acquire the first ultrasound imaging data while the processing unit is obtaining sensor data, or the first ultrasound imaging data may only be acquired at a same time (i.e. the determined time) as the second ultrasound imaging data.

In some examples, the processing system is configured to segment the anatomical structure in the region of interest by: generating a combined image of the region of interest and the extended field-of-view based on the first ultrasound imaging data and the second ultrasound imaging data; and segmenting the anatomical structure based on the combined image.

The use of a single (continuous) image as an input to a segmentation algorithm is the usual approach. The segmentation may therefore be performed simply using existing segmentation algorithms.

In some examples, the processing system is further configured to process the combined image to improve an image quality of the extended field-of-view region of the combined image. For instance, the processing system may use classical image processing algorithms or machine learning algorithms to improve the image quality. Enhancing the image quality of the extended field-of-view further improves an accuracy and reliability of the segmentation.

In some examples, the processing system is configured to segment the anatomical structure in the region of interest by: generating a first image based on the first ultrasound imaging data; generating a second image based on the second ultrasound imaging data; and segmenting the anatomical structure based on the first and second images.

Providing separate images of the region of interest and extended field-of-view as input to a segmentation algorithm, rather than using a combined image, reduces a likelihood of artifacts (which may arise near the border between the region of interest and the extended field-of-view) affecting the segmentation result.

In some embodiments, the processing system is configured to process the second image (e.g. using a classical image processing algorithm or a machine-learning algorithm to improve an image quality of the second image before segmenting the anatomical structure.

In some embodiments, the processing system is further configured to redefine the extended field-of-view based on the segmentation.

The extended field-of-view may be redefined to achieve a desired compromise between frame rate and the size of the extended field-of-view.

For example, it may be determined, based on the segmentation, that the extended field-of-view was larger than necessary for stabilizing the segmentation. The extended field-of-view may then be reduced to an area determined to be most important for segmentation, thus improving a frame rate of subsequently acquired ultrasound imaging data without significantly affecting the segmentation results.

There is also proposed an ultrasound system comprising: the processing system described above; and a beamforming unit, configured to: acquire the first ultrasound imaging data by transmitting a first plurality of transmit pulses to the region of interest; and acquire the second ultrasound imaging data by transmitting a second plurality of transmit pulses to the extended field-of-view.

In some examples, the first plurality of transmit pulses is a plurality of overlapping transmit pulses and the second plurality of transmit pulses is a plurality of non-overlapping transmit pulses.

This allows an extended field-of-view region that extends laterally from the region of interest to be imaged at a lower resolution that the region of interest.

In some examples, a diverging transmit beam focal distance for each of the second plurality of transmit pulses is smaller than a focal distance for each of the first plurality of transmit pulses.

This allows an extended field-of-view region that extends laterally from the region of interest to be imaged at a lower resolution that the region of interest. The focal distance may be decreased gradually from the center of the region of interest to the extended field-of-view to keep a number of transmit pulses constant.

In some examples, a time interval between two consecutive transmit pulses of the second plurality of transmit pulses is greater than a time interval between two consecutive transmit pulses of the first plurality of transmit pulses.

Increasing a time interval between transmit pulses increases a depth of the field-of-view. In this way, an extended field-of-view region that extends axially beyond the region of interest may be imaged.

According to another aspect of the invention, there is provided a computer-implemented method for segmenting an anatomical structure in an ultrasound image, the computer-implemented method comprising: obtaining first ultrasound imaging data representative of a region of interest including the anatomical structure, wherein the first ultrasound imaging data is acquired at a first resolution; obtaining second ultrasound imaging data representative of an extended field-of-view, wherein the second ultrasound imaging data is acquired at a second, lower resolution, and the extended field-of-view comprises the region of interest and at least one further region adjacent to the region of interest; and segmenting the anatomical structure in the region of interest by processing the first ultrasound imaging data and second ultrasound imaging data.

There is also proposed a computer program product comprising computer program code means which, when executed on a computer device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an exemplary ultrasound system;
Fig. 2 illustrates an ultrasound system, according to an embodiment of the invention;
Fig. 3 illustrates an example pulse sequence by which ultrasound imaging data of the region of interest and extended field-of-view may be acquired at different resolutions;
Fig. 4 illustrates an example of a high resolution ultrasound image of a region of interest;
Fig. 5 illustrates an example of a low resolution ultrasound image of an extended field-of-view comprising the region of interest in Fig. 4;
Fig. 6 illustrates an example of a combined image of a region of interest and extended field-of-view; and
Fig. 7 illustrates a computer-implemented method for segmenting an anatomical structure in ultrasound imaging data, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a system and method for segmenting an anatomical structure in ultrasound imaging data. First ultrasound imaging data of a first region, which includes the anatomical structure, is acquired at a high resolution. Second ultrasound imaging data of a second region, which includes a region neighboring the first region, is acquired at a low resolution. The first and second ultrasound imaging data are processed to segment the anatomical structure.

Embodiments are at least partly based on the realization that the region(s) around a small field-of-view imaged in an ultrasound imaging process would provide additional anatomical context that would aid the segmentation of anatomical objects in the small field-of-view, and that imaging the region(s) around the small field-of-view at a low resolution would provide sufficient information to improve segmentation without having a significant adverse effect on frame rate.

Illustrative embodiments may, for example, be employed in any ultrasound applications that typically have a trade-off between field-of-view size and temporal resolution and use a segmentation algorithm that would benefit from a larger field-of-view, such as three-dimensional ultrasound applications, including three-dimensional cardiac ultrasound.

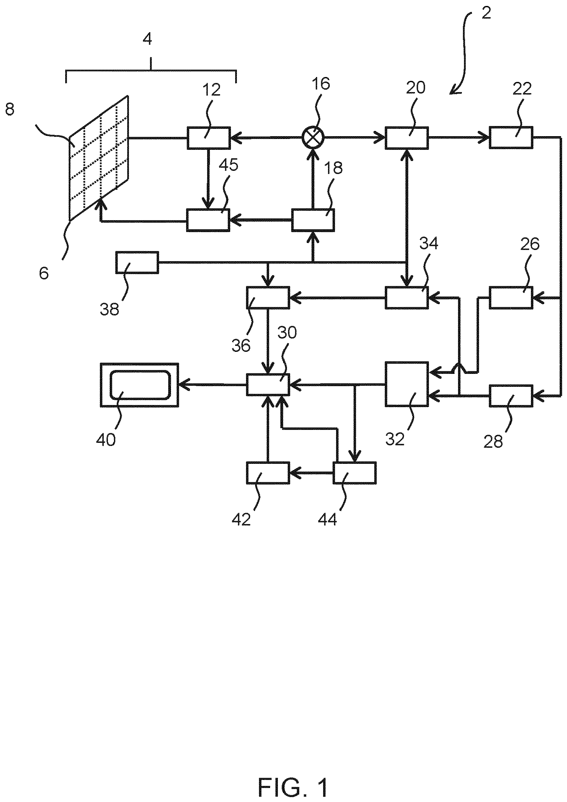

The general operation of an exemplary ultrasound system will first be described, with reference to Fig. 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

A special case is called diverging wave transmits (a.k.a. divergent beams), where the geometric focus of the beam is assumed behind the transducer. These type of transmit beams always diverge in the transmitted media. Such a beam can be emitted by transmitting the central element first and neighboring elements later towards the edges of the transducer, where the delays are computed based on the diverging focus geometry. Similar to focused beams, the diverging wave transmits can be also steered. Divergent waves are useful for insonifying a larger region of interest with limited number of transmits therefore suitable for fast imaging sequences and high frame rates.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

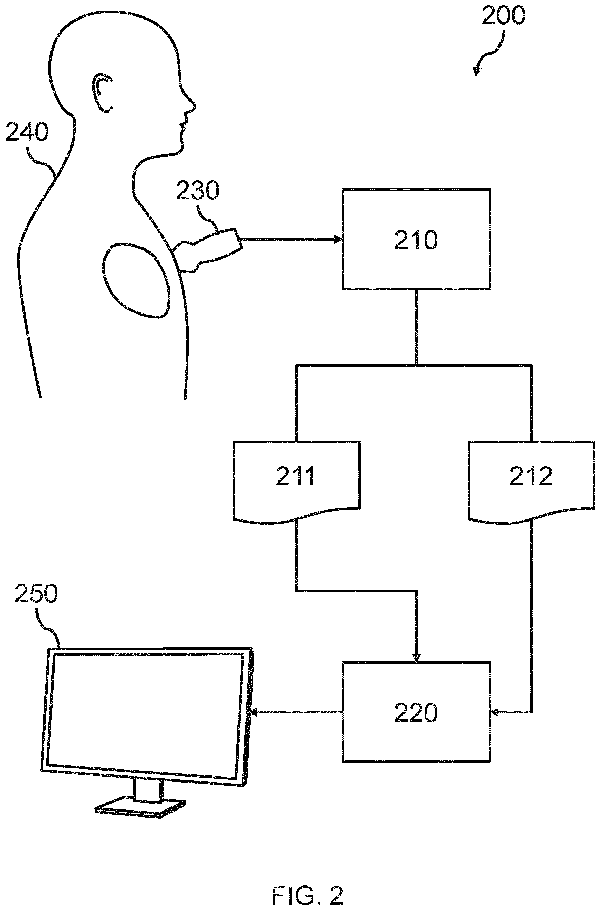

Fig. 2 illustrates an ultrasound system 200, according to an embodiment of the invention. The system comprises a beamforming unit 210 and a processing system 220 for segmenting an anatomical structure in ultrasound imaging data. The processing system is, itself, an embodiment of the invention.

The beamforming unit 210 is connected to an ultrasound transducer probe 230, and is configured to acquire, using the ultrasound transducer probe, first ultrasound imaging data 211 at a first resolution and second ultrasound imaging data 212 at a second, lower resolution. The beamforming unit and transducer probe may be a conventional ultrasound beamforming unit and transducer probe, as described above with reference to Fig. 1, adapted to acquire imaging data for different regions at different resolutions.

The axial and lateral resolution of the first ultrasound imaging data may be close to the axial and lateral resolution respectively of the second ultrasound imaging data, because the axial resolution is mainly defined by the transducer transmit wave bandwidth and the lateral resolution is defined by the transducer aperture size, which does not change. However, the second ultrasound imaging data may have much higher side-lobe levels due to the lack of transmit beam focus. Although the first ultrasound imaging data may also use divergent waves and lack geometric transmit focus, multiple overlapping transmits (e.g. n = 4, where n is a number of beams) can be coherently compounded to create a retrospective transmit focusing effect. The second ultrasound imaging data may not use any coherent compounding, or a small number of beams may be compounded (e.g. n = 1, 2). The result of coherent compounding is a reduction in the clutter levels in the image. According to Papadacci et al (2014), "High contrast ultrafast imaging of the human heart", IEEE Trans Ultrason Ferroelectr Freq Control, 61(2):288-301, the reduction in clutter levels by coherent compounding 4 divergent wave transmits compared to 2 divergent wave transmits is 3 dB and compared to no compounding (i.e. imaging a pixel with a single divergent beam) is 5 dB. If focused beams are used, clutter levels are 9 dB lower than the 4 divergent beam compounding case. The reduced clutter levels of the ultrasound image makes the segmentation algorithms run with better performance.

The first ultrasound imaging data 211 is imaging data representative of a region of interest including an anatomical structure to be segmented. In Fig. 2, the anatomical structure is an anatomical structure in a heart of a subject 240, but the ultrasound system 200 may be used to improve a segmentation of any anatomical structure for which ultrasound images generally have too small a field-of-view for reliable automatic segmentation due to a desired frame rate (that is, in any ultrasound applications having a trade-off between field-of-view size and temporal resolution for which a segmentation algorithm would benefit from a larger field-of-view, such as 3D cardiac ultrasound and 3D fetal ultrasound imaging). The region of interest is a region with a relatively small field-of-view that includes the anatomical structure. For example, the region of interest may be just large enough to contain the anatomical structure. The first ultrasound imaging data is acquired at a high resolution (i.e. a resolution suitable for display purposes).

The second ultrasound imaging data 212 is imaging data representative of an extended field-of-view that comprises at least one region adjacent to the region of interest. In some examples, the extended field-of-view may additionally comprise the region of interest and/or part of the region of interest. In other words, the extended field-of-view may be a larger region that includes (at least part of) the region of interest. The second ultrasound imaging data is acquired at a low resolution (i.e. a resolution sufficiently low to acquire imaging data of the extended field-of-view at a desired frame rate, but high enough to provide contextual information), as the second ultrasound imaging data is not intended for display, but to provide additional anatomical context for segmenting the anatomical structure.

In some examples, the first ultrasound imaging data 211 and second ultrasound imaging data 212 may be three-dimensional ultrasound imaging data, in which the trade-off between field-of-view size and temporal resolution is a particular problem. Thus, the proposed approach finds particular use and is of particular advantage in this use case scenario.

In some examples, the processing system 220 may obtain a definition of the region of interest and define the extended field-of-view based on the received definition of the region of interest. The definition of the region of interest may be obtained based on user input, received, for example, at a user interface 250.

The extended field-of-view may be defined according to one or more predetermined rules, which may depend on the anatomical structure to be segmented in the region of interest. For instance, the extended field-of-view may be defined such that the extended field-of-view is larger than the region of interest by a predefined percentage and contains the region of interest at its center. The predefined percentage may depend on the anatomical structure to be segmented; for example, where the anatomical structure is a mitral valve, the extended field-of-view may be 20-80% larger than the region of interest, e.g. 50% larger. The region of interest may be centered with respect to the extended field-of-view, but the extended field-of-view may also be asymmetrically arranged with respect to the region of interest (i.e. the extended field-of-view may extend from the region of interest further in a first direction than in a second, different direction).

The processing system 220 may be configured to control the beamforming unit 210 to acquire the first ultrasound imaging data 211 at the first resolution and to acquire the second ultrasound imaging data 212 at the second resolution.

In some examples, the processing system 220 may be configured to first control the beamforming unit 210 to acquire the first ultrasound imaging data 211, generate an image of the region of interest based on the first ultrasound imaging data and output the generated image of the region of interest to the user interface 250. The processing system may continue to obtain the first ultrasound imaging data and output the generated image of the region of interest until a user input is received (e.g. via the user interface) indicating that a current region of interest corresponds to a desired field-of-view. In other words, only the first ultrasound imaging data may be acquired while a user is adjusting the probe to find a desired field-of-view (showing a desired view of the anatomical structure). The user may then indicate that the current view displayed on the user interface is suitable for capturing via the user input.

In response to receiving the user input, the processing system 220 may then control the beamforming unit 210 to acquire the second ultrasound imaging data 212 in addition to the first ultrasound imaging data 212.

In other examples, the processing system 220 may be configured to automatically determine a time at which to acquire the second ultrasound imaging data 212. This may, for example, enable the second ultrasound imaging data to be acquired at a particular point in a cyclical motion of the anatomical structure (or of an anatomical object of which the anatomical structure is part), such as a particular point in the cardiac cycle (e.g. end-diastole or end-systole). In other words, the processing system may control the beamforming unit to acquire the second ultrasound imaging data only for predetermined frames in an ultrasound acquisition series (such as an end-diastolic and/or end-systolic frame in the case of cardiac ultrasound).

For instance, the processing system 220 may be configured to first control the beamforming unit 210 to acquire the first ultrasound imaging data 211 and determine a time at which to acquire the second ultrasound imaging data 212 based on the first ultrasound imaging data. The processing system may then control the beamforming unit to acquire the second ultrasound imaging data at the determined time.

The processing system 220 may determine a time at which to acquire the second ultrasound imaging data 212 based on the first ultrasound imaging data 211 by processing the first ultrasound imaging data to determine one or more time-dependent anatomical characteristics, and determining a time at which to acquire the second ultrasound imaging data based on the determined one or more time-dependent anatomical characteristics. The one or more time-dependent anatomical characteristics may be any suitable time-dependent characteristics of the anatomical structure, or other anatomical objects in the region of interest, that are capable of being determined from the first ultrasound imaging data, and may depend on the particular anatomical structure and/or region of interest.

For example, where the first ultrasound imaging data 211 and second ultrasound imaging data 212 are cardiac ultrasound imaging data, the one or more time-dependent anatomical characteristics determined from the first ultrasound imaging data may comprise at least one of a ventricular volume and/or an opening state of mitral leaflets.

Methods of determining anatomical characteristics from ultrasound imaging data are well known, and suitable methods for determining the one or more time-dependent characteristics will be apparent to the skilled person. In some examples, an initial segmentation may be performed on the first ultrasound imaging data (i.e. a segmentation without the additional anatomical context provided by the second ultrasound imaging data) in order to determine the one or more time-dependent characteristics. In other examples, a specialized image processing algorithm that does not provide a complete segmentation but estimates one or more time-dependent characteristics based on ultrasound imaging data (e.g. an AI-based cardiac phase detector) may be used to determine the one or more time-dependent characteristics.

In some examples, the system 200 may further comprise one or more sensors (not shown in Fig. 2) capable of receiving sensor data representative of the anatomical structure. The processing 220 may be configured to obtain sensor data from the one or more sensors and determine a time at which to acquire the second ultrasound imaging data 212 based on the sensor data. The processing system may then control the beamforming unit to acquire the second ultrasound imaging data at the determined time. In these examples, the processing system may control the beamforming unit to acquire the first ultrasound imaging data 211 throughout the process (i.e. the beamforming unit acquires the first ultrasound imaging data while the processing system is obtaining and processing the sensor data and continues to acquire the first ultrasound imaging data while acquiring the second ultrasound imaging data), or the first ultrasound imaging data may also be acquired only at the determined time.

The processing system 220 may determine a time at which to acquire the second ultrasound imaging data 212 based on the sensor data by processing the sensor data to determine one or more time-dependent anatomical characteristics, and determining a time at which to acquire the second ultrasound imaging data based on the determined one or more time-dependent anatomical characteristics. Similarly to the anatomical characteristics determined from the first ultrasound imaging data 211, the one or more time-dependent anatomical characteristics determined from the sensor data may be any suitable time-dependent characteristics of the anatomical structure, or other anatomical objects in the region of interest, that are capable of being determined from the sensor data, and may depend on the particular anatomical structure and/or region of interest and on the type of sensor(s) used to obtain the sensor data.

Suitable sensors for obtaining the sensor data and methods for determining the one or more time-dependent characteristics based on the sensor data will be apparent to the skilled person. For instance, the one or more sensors may comprise one or more of: an ECG (electrocardiography) sensor, a PCG (phonocardiography) sensor, a pulse sensor, and/or a camera (for camera-based heart rate monitoring). In particular, ECG sensors are commonly used to determine information relating to a cardiac cycle of a subject. The one or more time-dependent characteristics may, for example, comprise an amplitude of an ECG trace and/or an amplitude of a PCG trace.

The beamforming unit 210 acquires the first ultrasound imaging data 211 by transmitting a first plurality of transmit pulses to the region of interest, and acquires the second ultrasound imaging data by transmitting a second plurality of transmit pulses to the extended field-of-view.

The first plurality of transmit pulses may be a conventional ultrasound imaging acquisition sequence. For example, the first plurality of transmit pulses may comprise spatially thin ultrasound beams with an overlap between transmit beams.

Where the extended field-of-view extends laterally from the region of interest (i.e. extends in a direction perpendicular to a direction of the first plurality of transmit pulses), the second plurality of transmit pulses may, for example, comprise non-overlapping transmit beams transmitted to at least one side of the region of interest. The at least one side to which the non-overlapping transmit beams are transmitted will depend on the definition of the extended field-of-view.

Additionally or alternatively, the second plurality of transmit pulses may be broader (i.e. compared to the first plurality of transmit pulses) divergent beams formed by moving the focal point behind the transducer aperture closer to create larger coverage. In other words, a diverging transmit beam focal distance (that is, a focal distance of a divergent beam) for each of the second plurality of transmit pulses may be smaller than a focal distance for each of the first plurality of transmit pulses.

The total number of transmit pulses may be kept constant between acquiring only the first ultrasound imaging data 211 and acquiring both the first ultrasound imaging data and the second ultrasound imaging data 212 by gradually decreasing the focal distance of the first plurality of transmit pulses towards the edges of the region of interest, and gradually decreasing the focal distance of the second plurality of transmit pulses as the distance from the region of interest increases.

Where the extended field-of-view extends axially from the region of interest (i.e. extends in a direction of the first plurality of transmit pulses), the second plurality of transmit pulses may have a longer time interval between two consecutive transmit pulses than the first plurality of transmit pulses. Increasing a time between two consecutive transmit pulses and receiving backscattered echoes from the transmit pulses for longer allows a depth of an imaging field-of-view to be increased.

To reduce the effect of increasing a time interval between two consecutive transmit pulses on frame rate, the time interval may not be increased for every transmit. For example, the time interval between two consecutive transmit pulses may be greater for every other transmit. In an example in which the transmit pulses comprises four overlapping beams, a time interval between two consecutive transmit pulses may be greater for every fourth transmit. Alternatively, a gradual or cyclic approach to varying the time interval may be used. If the region of interest ends at a depth of z₀ and the extended field-of-view at a depth of z₀ + 2Δz, the time intervals between consecutive transmit pulses for four transmit pulses may be adjusted such that a first transmit pulse ends at z₀, a second transmit pulse and a fourth transmit pulse each end at z₀ + Δz, and a third transmit pulse ends at z0 + 2Δz. In this way, a resolution of the first ultrasound imaging data will remain sufficiently high, but a resolution of the second ultrasound imaging data will decrease between z₀ and z₀ + Δz and between z₀ + Δz and z₀ + Δ2z.

In other words, where the extended field-of-view extends from the region of interest in an axial direction only, the first and second plurality of transmit pulses may be considered as a single sequence of transmit pulses, with a longer time interval between some consecutive transmit pulses than between others.

In some examples, the extended field-of-view may extend from the region of interest both laterally and axially. In these examples, a combination of non-overlapping pulses and/or smaller focal distance(s) and greater time interval(s) may be used for the second plurality of transmit pulses to image regions of the extended field-of-view that extend from the region of interest in the lateral direction and regions that extend in both the axial and lateral directions (i.e. that extend axially and are outside the lateral extent of the region of interest). The first plurality of transmit pulses may have a longer time interval between some consecutive transmit pulses than between other transmit pulses, in order to image regions of the extended field-of-view that extend from the region of interest in the axial direction only.

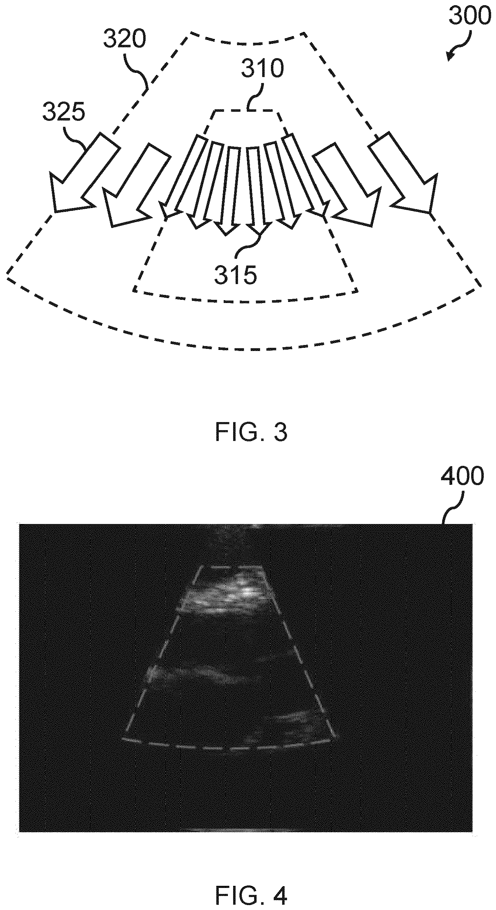

Fig. 3 illustrates an example pulse sequence 300 by which ultrasound imaging data of the region of interest and extended field-of-view may be acquired at different resolutions.

According to the pulse sequence 300 in Fig. 3, the first ultrasound imaging data 211 is acquired by transmitting the first plurality of transmit pulses 315 to the region of interest 310. The first plurality of transmit pulses comprise narrow beams with a high line density.

The second ultrasound imaging data 212 is acquired by transmitting the second plurality of transmit pulses 325 to the extended field-of-view 320. The second plurality of transmit pulses comprise wider transmit beams with a lower line density than the first plurality of transmit pulses 315, and are transmitted each side of the first plurality of transmit pulses to extend the field-of-view laterally. In other words, the second plurality of transmit pulses in Fig. 3 uses both of the methods described above for extending the field-of-view laterally (i.e. non-overlapping transmit beams and broader, divergent beams).

Returning to Fig. 2, the processing system 220, having obtained the first ultrasound imaging data 211 and second ultrasound imaging data 212, processes the first and second ultrasound imaging data to segment the anatomical structure in the region of interest. By using the second ultrasound imaging data as well as the first ultrasound imaging data, the segmentation may be based on additional anatomical context which would not be available from the first ultrasound imaging data alone, thus improving an accuracy and reliability of the segmentation result.

The processing system 220 may segment the anatomical structure using any suitable segmentation algorithm, including voxel-based segmentation algorithms, mesh-model-based segmentation algorithms and AI-based segmentation algorithms (e.g. using one or more convolutional neural networks or other machine-learning methods). The segmentation of the anatomical structure involves the identification of the boundaries of the anatomical structure. In performing the segmentation, the processing system may also segment/identify one or more other anatomical objects in the region of interest and/or the extended field-of-view.

In some examples, the processing system 220 may process the first ultrasound imaging data 211 to generate a first image (i.e. a high resolution image of the region of interest) and processes the second ultrasound imaging data 212 to generate a second image (i.e. a low resolution image of the extended field-of-view). Where the extended field-of-view comprises the region of interest, the second image may be an image showing the entire extended field-of-view (i.e. including the region of interest) or an image showing only the at least one region adjacent to the region of interest (i.e. the region of interest may be omitted from the second image).

The segmentation algorithm may then take the first and second images as input for the segmentation. For instance, in the case of a model-based segmentation algorithm, the segmentation algorithm may detect target points in both the first and second images. In the case of an AI-based segmentation algorithm, the first and second images may be received as different input channels. Further examples of how a segmentation algorithm may segment an anatomical structure based on a high resolution image including the anatomical structure and a low resolution image providing further anatomical context will be apparent to the skilled person.

In some examples, an image quality of the second image may be improved before the segmentation algorithm is applied. Improving an image quality of the second image may include one or more of improving a resolution, improving a contrast, and/or reducing speckle, clutter, noise, and/or artifacts.

The image quality of the second image may, for example, be improved using one or more classical image processing algorithms and/or one or more machine-learning algorithms. In some examples, the first image may be used to improve the image quality of the second image. For instance, the first image may be input into a machine-learning algorithm used to improve an image quality of the second image. In some examples, multiple algorithms to improve image quality may be applied to the second image, where each algorithm improves an image quality differently (e.g. a first algorithm may be used to improve a resolution and a second algorithm may be used to reduce speckle).

Methods for improving image quality of ultrasound images, including machine-learning methods, are well known and suitable methods will be apparent to the skilled person. See, for example, Kokil and Sudharson (2020), "Despeckling of clinical ultrasound images using deep residual learning", Comput Methods Programs Biomed. 194:105477, Perdios et al. (2018), "Deep convolutional neural network for ultrasound image enhancement", 2018 IEEE International Ultrasonics Symposium (IUS), pp. 1-4, and Abdel-Nasser and Omer (2017), "Ultrasound image enhancement using a deep learning architecture", Proceedings of the International Conference on Advanced Intelligent Systems and Informatics 2016, AISI 2016, Advances in Intelligent Systems and Computing, vol. 533, Springer.

Fig. 4 illustrates a high resolution ultrasound image 400 of a region of interest. Image 400 is a parasternal long axis view of the heart of a subject, showing the left and right ventricles, septum and heart valves. This image may be displayed to a clinician (e.g. via user interface 250).

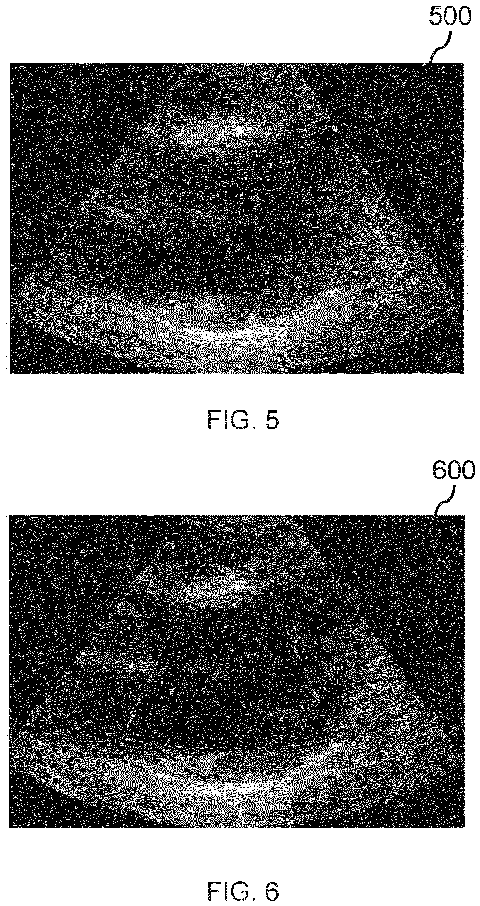

Fig. 5 illustrates a low resolution ultrasound image 500 of an extended field-of-view comprising the region of interest in Fig. 4, acquired using the methods described above. This image may provide additional context for segmenting one or more of the anatomical structures shown in Fig. 4.

Images 400 and 500 may be input into a segmentation algorithm as described above in order to segment the anatomical structure(s). In particular, these images may be used to segment the left ventricle, in order to determine a left ventricular volume. An image quality of image 400 may be improved as described above before being input into the segmentation algorithm.

Returning to Fig. 2, in some examples, the processing system 220 may process the first ultrasound imaging data 211 and the second ultrasound imaging data 212 to generate a combined image of both the region of interest and the extended field-of-view, where the region of the combined image corresponding to the region of interest has a high resolution and the remainder of the combined image (i.e. the region corresponding to the at least one region of the extended field-of-view) has a low resolution.

The segmentation algorithm may then take the combined image as input for the segmentation. For instance, in the case of a model-based segmentation algorithm, vertices of a mean mesh may be adapted to the entire image, including the region of the combined image corresponding to the at least one region of the extended field-of-view. In the case of an AI-based segmentation algorithm, an output of the segmentation algorithm may comprise a classification of each image voxel. Further examples of how a segmentation algorithm may segment an anatomical structure based on such a combined image will be apparent to the skilled person.

In some examples, an image quality of the extended field-of-view region of the combined image may be improved before the segmentation algorithm is applied. Improving an image quality of the extended field-of-view region may, as described above with reference to the second image, include one or more of improving a resolution, improving a contrast, and/or reducing speckle, clutter and/or artifacts.

As described above with reference to the second image, the image quality of the extended field-of-view region of the combined image may, for example, be improved using one or more classical image processing algorithms and/or one or more machine-learning algorithms. In some examples, the region of interest may be used to improve the image quality of the extended field-of-view region of the combined image. For instance, the region of the combined image corresponding to the region of interest may be input into a machine-learning algorithm used to improve an image quality of the extended field-of-view region of the combined image.

Improving an image quality of the extended field-of-view region of the combined image may additionally or alternatively include smoothing a transition region close to a boundary between the region of interest and the at least one region of the extended field-of-view to reduce artificial image gradients. For instance, a Gaussian blurring filter with radius r may be applied to the border of the region of interest. A size of the transition region may depend on a size of the region of interest and a difference between the first resolution (i.e. the resolution of the region of interest) and the second resolution (i.e. the resolution of the extended field-of-view), and may be predetermined or determined by the processing system (e.g. based on predetermined rules).

Fig. 6 illustrates a combined image 600 of a region of interest and extended field-of-view, in which the region of interest has a high resolution and the region outside the region of interest has a lower resolution. Image 600 may be considered as a combination of image 400 of Fig. 4 and image 500 of Fig. 5.

Image 600 may be input into a segmentation algorithm as described above in order to segment the anatomical structure. An image quality of the extended field-of-view region of image 600 may be improved as described above before being input into the segmentation algorithm.

Having segmented the anatomical structure in the region of interest, the processing system 220 may output an image of the region of interest and a representation of the segmentation result to the user interface 250 for display to a clinician. Methods of displaying a segmentation of an anatomical structure are well known and will be apparent to the skilled person. For example, the processing system may overlay a boundary of the anatomical structure in the image of the region of interest on the image.

The processing system 220 may have generated an image of the region of interest prior to segmentation (the first image generated based on the first ultrasound imaging data 211 described above). In this case, the processing system 220 may output the already-generated image to the user interface 250. Where a combined image of the region of interest and the extended field-of-view was used to segment the anatomical structure, the processing system may generate an additional image showing the region of interest only, by processing the first ultrasound imaging data. This avoids displaying the low resolution region of the combined image.

In some examples, it may be desirable to generate a series of ultrasound images over time (i.e. a series of images of the region of interest). For example, in the case of cardiac ultrasound imaging, it may be desirable to generate a series of ultrasound images over the cardiac cycle. In these cases, the first image of the region of interest in the series may be generated and segmented as described above, and the results of the segmentation may be used in the acquisition and/or segmentation of subsequent images of the region of interest in the series.

For instance, the results of the segmentation may be used to determine a time at which second ultrasound imaging data 212 is to be acquired for segmenting the anatomical structure in a subsequent (i.e. second or later) image in a series of images of the region of interest. In the case of cardiac ultrasound imaging, for example, at least one cardiac volume may be determined based on the results of the segmentation, and a time of an end-diastole and/or end-systole may be determined based on the determined cardiac volume. A time at which to acquire second ultrasound imaging data (e.g. in order to obtain an image of the extended field-of-view at end-diastole and/or end-systole) may then be determined based on the determined time of end-diastole and/or end-systole.

In some examples, having segmented the anatomical structure for a first image of the region of interest, the processing system 220 may re-define the extended field-of-view based on the segmentation result. For example, the processing system 220 may reduce a size of an extended field-of-view such that a frame rate is improved while maintaining a desired accuracy and reliability of the segmentation. The processing system may re-define the extended field-of-view using one or more predetermined rules.

For instance, the extended field-of-view may be re-defined such that the extended field-of-view comprises the anatomical structure (as identified in the segmentation) and a margin around the anatomical structure. The size of the margin may be predetermined according to the particular anatomical structure being segmented, such that the margin will define a region around the anatomical structure sufficiently large to provide a desired level of anatomical context for the anatomical structure. In another example, the extended field-of-view may be redefined such that the extended field-of-view comprises the anatomical structure (as identified in the segmentation) and one or more other anatomical objects identified in the segmentation. Again, the one or more other anatomical objects may be predetermined according to the particular anatomical structure being segmented.

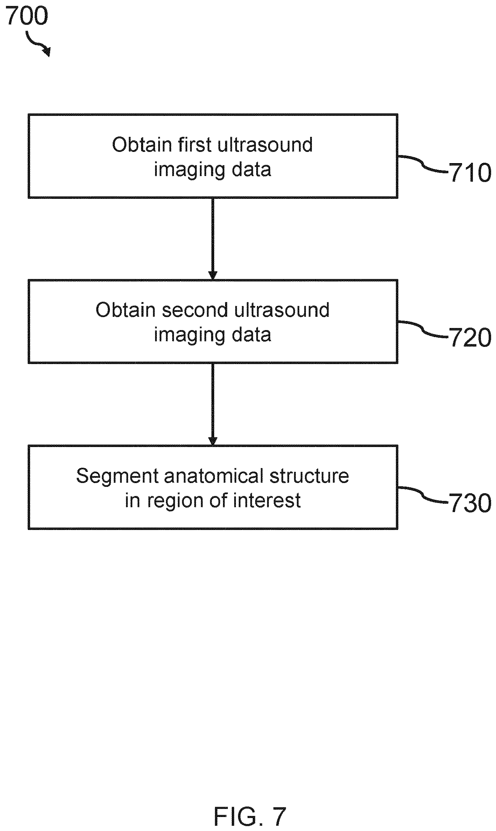

Fig. 7 illustrates a computer-implemented method 700 for segmenting an anatomical structure in ultrasound imaging data, according to an embodiment of the invention.

The method begins at step 710, at which first ultrasound imaging data representative of a region of interest including the anatomical structure is obtained. The first ultrasound imaging data is acquired at a first resolution.

At step 720, second ultrasound imaging data representative of an extended field-of-view is obtained. The extended field-of-view comprises at least one region adjacent to the region of interest. The second ultrasound imaging data is acquired at a second, lower resolution.

At step 730, the first ultrasound imaging data and second ultrasound imaging data are processed to segment the anatomical structure in the region of interest.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and

EEPROM. The storage media may be encoded with one or more programs that, when executed on one or more processors and/or controllers, perform the required functions. Various storage media may be fixed within a processor or controller may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A processing system (220) for segmenting an anatomical structure in ultrasound imaging data, the processing system being configured to:
obtain first ultrasound imaging data (211) representative of a region of interest (310) including the anatomical structure, wherein the first ultrasound imaging data is acquired at a first resolution;
obtain second ultrasound imaging data (212) representative of an extended field-of-view (320), wherein the second ultrasound imaging data is acquired at a second, lower resolution, and the extended field-of-view comprises at least one region adjacent to the region of interest; and
segment the anatomical structure in the region of interest by processing the first ultrasound imaging data and second ultrasound imaging data.

2. The processing system (220) of claim 1, wherein the processing system is further configured to:
obtain a definition of the region of interest (310); and
define the extended field-of-view (320) based on the received definition of the region of interest.

3. The processing system (220) of claim 1 or 2, wherein the processing system is further configured to control a beamforming unit (210) to:
acquire first ultrasound imaging data (211) representative of the region of interest (310) at the first resolution; and
acquire second ultrasound imaging data (212) representative of the extended field-of-view (320) at the second, lower resolution.

4. The processing system (220) of claim 3, wherein the processing system is configured to:
control the beamforming unit (210) to acquire the first ultrasound imaging data (211);
generate an image of the region of interest (310) based on the first ultrasound imaging data;
output the generated image of the region of interest to a user interface (250); and
control the beamforming unit to acquire the second ultrasound imaging data (212) in response to receiving a user input.

5. The processing system (220) of claim 3, wherein the processing system is configured to:
control the beamforming unit (210) to acquire the first ultrasound imaging data (211);
process the first ultrasound imaging data to determine one or more time-dependent anatomical characteristics;
determine a time at which to acquire the second ultrasound imaging data (212) based on the determined one or more time-dependent anatomical characteristics; and
control the beamforming unit to acquire the second ultrasound imaging data at the determined time.

6. The processing system (220) of claim 3, wherein the processing system is configured to:
obtain, from one or more sensors, sensor data representative of the anatomical structure;
process the sensor data to determine one or more time-dependent anatomical characteristics;
determine a time at which to acquire the second ultrasound imaging data (212) based on the determined one or more time-dependent anatomical characteristics; and
control the beamforming unit (210) to acquire the second ultrasound imaging data at the determined time.

7. The processing system (220) of any of claims 1 to 6, wherein the processing system is configured to segment the anatomical structure in the region of interest (310) by:
generating a combined image (600) of the region of interest and the extended field-of-view (320) based on the first ultrasound imaging data (211) and the second ultrasound imaging data (212); and
segmenting the anatomical structure based on the combined image.

8. The processing system (220) of any of claims 1 to 6, wherein the processing system is configured to segment the anatomical structure in the region of interest (310) by:
generating a first image (400) based on the first ultrasound imaging data (211);
generating a second image (500) based on the second ultrasound imaging data (212); and
segmenting the anatomical structure based on the first and second images.

9. The processing system (220) of any of claims 1 to 8, wherein the processing system is further configured to redefine the extended field-of-view (320) based on the segmentation.

10. An ultrasound system (200) comprising:
the processing system (220) of any of claims 1 to 9; and
a beamforming unit (210), configured to:
acquire the first ultrasound imaging data (211) by transmitting a first plurality of transmit pulses (315) to the region of interest (310); and
acquire the second ultrasound imaging data (212) by transmitting a second plurality of transmit pulses (325) to the extended field-of-view (320).

11. The ultrasound system (200) of claim 10, wherein the first plurality of transmit pulses (315) is a plurality of overlapping transmit pulses and the second plurality of transmit pulses (325) is a plurality of non-overlapping transmit pulses.

12. The ultrasound system (200) of claim 10 or 11, wherein a diverging transmit beam focal distance for each of the second plurality of transmit pulses (325) is smaller than a focal distance for each of the first plurality of transmit pulses (315).

13. The ultrasound system (200) of any of claims 10 to 12, wherein a time interval between two consecutive transmit pulses of the second plurality of transmit pulses (325) is greater than a time interval between two consecutive transmit pulses of the first plurality of transmit pulses (315).

14. A computer-implemented method (700) for segmenting an anatomical structure in an ultrasound image, the computer-implemented method comprising:
obtaining first ultrasound imaging data (211) representative of a region of interest (310) including the anatomical structure, wherein the first ultrasound imaging data is acquired at a first resolution;
obtaining second ultrasound imaging data (212) representative of an extended field-of-view (320), wherein the second ultrasound imaging data is acquired at a second, lower resolution, and the extended field-of-view comprises at least one region adjacent to the region of interest; and
segmenting the anatomical structure in the region of interest by processing the first ultrasound imaging data and second ultrasound imaging data.

15. A computer program product comprising computer program code means which, when executed on a computer device having a processing system, cause the processing system to perform all of the steps of the method (700) according to claim 14.
